# EUROPÄISCHE PATENTSCHRIFT

(11) **EP 1 733 678 B1**
(45) Veröffentlichungstag und Bekanntmachung des Hinweises auf die Patenterteilung: **29.02.2012**
(21) Anmeldenummer: 06010874.3
(22) Anmeldetag: 26.05.2006
(51) Int. Cl.: A61B 5/145, G06F 19/00

(54) **Glucoseanalysegerät mit Signalvorrichtung**
Glucose measurement apparatus with signalling device
Dispositif de mesure de glucose avec moyen de signalisation

(30) Priorität: 18.06.2005 EP 05013177
(43) Veröffentlichungstag der Anmeldung: 20.12.2006
(73) Patentinhaber: Roche Diagnostics GmbH, 68305 Mannheim (DE); F.Hoffmann-La Roche AG, 4070 Basel (CH)
(72) Erfinder: Werner, Karl, 69168 Wiesloch (DE); Stephan, Peter, 68167 Mannheim (DE); Lorenz, Robert, 67547 Worms (DE)
(74) Vertreter: Pfeifer, Hans-Peter

(56) Entgegenhaltungen:
- WO-A-01/74229
- WO-A-2006/037802
- US-A1- 2002 072 858
- US-A1- 2003 125 612
- US-A1- 2003 163 789
- US-A1- 2003 175 806

## Beschreibung

Die Erfindung betrifft ein Glucoseanalysegerät für Diabetiker mit einer Signaleinrichtung zum Erzeugen eines Erinnerungssignals.

Insbesondere richtet sich die Erfindung auf kleine, batteriebetriebene, tragbare Glucoseanalysegeräte, wie sie von Diabetikern im Rahmen der Blutzuckerselbstkontrolle (self-monitoring) verwendet werden. Solche Analysegeräte dienen zur Auswertung einer Reaktion, die in einem (auch als "Biosensor" oder "Teststreifen" bezeichneten) Analyseelement abläuft, nachdem es mit einem kleinen Tropfen der Probenflüssigkeit in Kontakt gebracht wurde. Die Probenflüssigkeit wird durch einen Stich in die Haut, insbesondere in eine Fingerkuppe, gewonnen. Abhängig von der Tiefe des Stichs besteht die Probenflüssigkeit normalerweise aus einem Gemisch von Blut und interstitieller Flüssigkeit aus der Haut. Obwohl demzufolge kein reines Blut untersucht wird, ist es gebräuchlich, von "Blutgloseanalysesystemen" zu sprechen. Die Erfindung richtet sich insbesondere auf derartige (für das self-monitoring gebräuchliche, sogenannte invasive) Systeme.

Glucoseanalysegeräte, die zusätzlich eine Signaleinrichtung zum Erzeugen eines Erinnerungssignals aufweisen, sind unter der Marke "Accu-Check Go" im Handel erhältlich. Die Signaleinrichtung des vorbekannten Glucoseanalysegeräts Accu-Check Go kann bis zu drei Tageszeiten speichern, zu denen der Benutzer durch einen Signalton an eine anstehende Blutzuckermessung erinnert wird. Die entsprechenden Tageszeiten werden von dem Benutzer gewählt und über Tasten in das Gerät eingegeben.

Da Glucoseanalysegeräte von Diabetikern ständig mitgeführt werden müssen, besteht die Forderung, sie möglichst kompakt zu gestalten. Dies hat zur Folge, dass Bedienungselemente wie Tasten oder Ähnliches nur in sehr kleiner Form und geringer Anzahl zur Verfügung gestellt werden können. Das Eingeben von Tageszeiten, zu denen durch einen Signalton an eine Blutzuckermessung erinnert werden soll, ist deshalb schwierig und mühsam. Dies trifft insbesondere auf Personen zu, deren manuelle Geschicklichkeit durch Alter oder Krankheit eingeschränkt ist.

In der US 2003/0175806 A1 wird ein Blutglucosemessgerät beschrieben, das im Verhältnis zu gebräuchlichen Geräten eine wesentlich häufigere Bestimmung der Glucosekonzentration dadurch ermöglichen soll, dass die Messung an alternativen Messorten stattfindet (Alternative Site Testing, AST). Das Messverfahren arbeitet reagenzienfrei, vorzugsweise nichtinvasiv. In diesem Zusammenhang wird die Möglichkeit beschrieben, zusätzliche Messungen mittels einer konventionellen invasiven ("finger-stick") Analyse durchzuführen und den Benutzer mehrfach am Tag an die Durchführung einer Blutanalysemessung zu erinnern. Gemäß einer in diesem Zusammenhang beschriebenen Variante wird die Erinnerungszeit nicht (wie bei Accu-Check Go) fest eingegeben, sondern unter Berücksichtigung des aktuellen Glucosewertes, eines Soll-Glucosewertes und gemessener Maximal- und Minimalwerte der gemessenen Konzentration ermittelt. Das Gerät speichert eine "case history" gemessener Konzentrationswerte, aus denen ein Prozessor des Gerätes die Erinnerungszeiten mittels mathematischer Vorhersageverfahren (z.B. mittels "Fuzzy-Logik") ermittelt.

Aufgabe der Erfindung ist es, einen verbesserten Weg aufzuzeigen, wie Benutzer zu individuell passenden Zeiten an das Durchführen einer Blutzuckermessung erinnert werden können.

Diese Aufgabe wird gelöst durch ein Glucoseanalysegerät gemäß Anspruch 1.

Die im Rahmen der Erfindung in dem Datenspeicher des Gerätes gespeicherten Ereigniszeitdaten beziehen sich auf vorab festgelegte (vordefinierte) Ereignisstypen im Tagesablauf des Benutzers. Vordefinierte Ereignistypen können insbesondere Messungen der Glucosekonzentration, Nahrungsaufnahmen, Ruhezeiten und Zeiten erhöhter körperlicher Aktivität sein. Grundsätzlich können aber auch andere Ereignisse, die für den Tagesablauf eines Benutzers charakteristisch sind, beispielsweise Beginn und Ende der Arbeitszeit, als Ereignistypen definiert und die entsprechenden Ereigniszeitdaten gespeichert werden.

Dabei sind zwei Klassen von Ereignistypen zu unterscheiden, nämlich erstens Ereignistypen, die sich auf eine Funktion des Glucoseanalysegerätes beziehen, so dass Informationen darüber in dem Analysegerät ohne weiteres zur Verfügung stehen ("interne Ereignisse") und zweitens Ereignistypen, die für Besonderheiten des Tagesablaufs des Benutzers kennzeichnend sind und sich nicht auf Funktionen des Glucoseanalysegerätes beziehen, so dass Informationen darüber in dem Gerät nicht ohne weiteres (ohne zusätzliche Mittel, z. B. Sensoren) zur Verfügung stehen ("externe Ereignisse"). Die in dem Datenspeicher des Gerätes gespeicherten Ereigniszeitdaten enthalten jeweils eine Information über die Zeit, zu der ein Ereignis, das einem vordefinierten Ereignistyp entspricht, stattfindet, sowie eine Information über den jeweiligen Ereignistyp.

Die abgespeicherten Ereigniszeiten sind typischerweise die Tageszeiten, zu denen das jeweilige Ereignis stattfindet. Bei länger andauernden Vorgängen, wie einer Nahrungsaufnahme oder einer Ruheperiode, kann als typische Tageszeit beispielsweise deren Beendigung oder der Mittelpunkt des Zeitraums, über den sich der Vorgang erstreckt, abgespeichert werden. Selbstverständlich ist die Erfindung jedoch nicht an eine bestimmte Form der Erfassung und Speicherung der zu den Ereigniszeiten gehörenden Zeitinformation gebunden. Diese Zeitinformation muss insbesondere nicht notwendigerweise als Tageszeit gespeichert werden. Vielmehr kann es zweckmäßig sein, einen zeitlichen Abstand, also die Zeitdauer zwischen zwei Ereignissen zu erfassen und abzuspeichern, ohne dass die absolute Zeit (Tageszeit und Datum), zu der die Ereignisse stattfinden, gespeichert oder auch nur erfasst werden muss.

Insbesondere kann die Zeitdauer, die zwischen zwei Ereignissen unterschiedlicher Ereignistypen liegt, erfasst und abgespeichert werden. Beispielsweise können die Ereigniszeitdaten den zeitlichen Abstand zwischen einer Nahrungsaufnahme und einer Glucosemessung umfassen. In diesem Fall basiert die Ermittlung der Erinnerungszeit (durch den Erinnerungszeitermittlungsalgorithmus) auf dem zeitlichen Abstand zwischen den Ereignissen der beiden Ereignistypen, ohne dass dabei die Tageszeiten, zu denen die Ereignisse eingetreten sind, berücksichtigt werden müssen. Eine solche Möglichkeit wird weiter unten im Zusammenhang mit der Verwendung einer Ereigniszeit als nicht statischen Bezugspunkt (floating reference point) erläutert.

Für den Ereignistyp "Messung einer Glucosekonzentration" wird jeweils die Zeit, zu der eine Analyse durchgeführt wird, aufgezeichnet. Selbstverständlich kann zusätzlich auch das Analyseergebniss, also die gemessene Konzentration, abgespeichert werden. Im Rahmen des Erinnerungszeitermittlungsalgorithmus wird vorzugsweise jedoch nur der Zeitpunkt der Durchführung einer Glucoseanalyse, nicht das Analyseergebnis, berücksichtigt.

Die Information darüber, dass ein einem externen Ereignistyp entsprechendes Ereignis stattfindet, kann von dem Benutzer manuell über eine Eingabeeinheit des Gerätes (z.B. mittels Tasten) eingegeben werden. Das Gerät kann jedoch auch einen Ereignissensor aufweisen, durch den das Ereignis automatisch erkannt und der entsprechende Ereigniszeit abgespeichert wird. Verhältnismäßig einfache und kostengünstige Sensoren sind vor allem zur Erfassung der körperlichen Aktivität verfügbar und werden beispielsweise in Schrittzähler eingebaut. Damit lassen sich sowohl Zeiten erhöhter körperlicher Aktivität als auch Ruhezeiten automatisch erkennen.

Im Vergleich zu einem Gerät mit manueller Eingabe der Erinnerungszeiten zeichnet sich das erfindungsgemäße Glucoseanalysegerät durch eine vereinfachte Bedienung und dadurch aus, dass die Erinnerungszeiten automatisch und flexibel an den Tagesablauf des Benutzers angepasst werden. Das in der US 2003/0175806 A1 beschriebene Verfahren ermöglicht zwar ebenfalls eine automatische Erzeugung von Erinnerungssignalen, jedoch passen sich diese nicht an den individuellen Tagesablauf des Benutzers an. Vielmehr wird dort der Versuch gemacht, Erinnerungszeiten auf Basis analytischer Ergebnisse zu ermitteln. Im Rahmen der Erfindung wurde festgestellt, dass dies ungeeignet ist, weil die Erinnerungszeit auf der Vorhersage der erwarteten weiteren Entwicklung der Glucosekonzentration basiert, die mit großen Unsicherheiten behaftet ist.

Bei einem erfindungsgemäßen Glucoseanalysegerät ist es nicht erforderlich, dass irgendwelche Tageszeiten von einem Benutzer eingegeben werden. Aus gespeicherten Daten über das bisherige Benutzerverhalten ermittelt die Auswerteeinrichtung selbsttätig die Zeiten, zu denen von der Signaleinrichtung ein Erinnerungssignal erzeugt wird.

Die Erinnerungsfunktion des Geräts passt sich von selbst an den individuellen Tagesablauf und das Verhalten eines Benutzers an. Besonders vorteilhaft ist, dass sich die Erinnerungsfunktion selbsttätig an eine Änderung des Tagesrhythmus anpasst, die beispielsweise jahreszeitlich bedingt sein kann. Die Erinnerungsfunktion eines erfindungsgemäßen Geräts stellt deshalb ein selbstlernendes Alarmsystem dar, durch das der Benutzer automatisch an Messungen der Blutglucosekonzentration und eventuell weitere zur Behandlung von Diabetes wichtige Maßnahmen erinnert wird. Die Erinnerungszeiten, zu denen die Signaleinrichtung betätigt wird, können beispielsweise durch Anwendung eines Mustererkennungsverfahrens aus den gespeicherten Ereigniszeitdaten ermittelt werden.

Zum Ermitteln der Erinnerungszeiten wird mittels der Steuer- und Auswerteeinrichtung mindestens ein Teil der gespeicherten Ereigniszeitdaten ausgewertet, wobei die für die Auswertung verwendeten Ereigniszeitdaten über mehrere Tage verteilt sind. Vorzugsweise werden Ereigniszeitdaten für mindestens 5, bevorzugt mindestens 8, besonders bevorzugt mindestens 12 Tage in Form von Tagesablaufprofildaten akkumuliert und mindestens eine Teilmenge dieser Gesamtdatenmenge in dem Erinnerungszeitermittlungsalgorithmus bei der Ermittlung einer Erinnerungszeit verwendet.

Ein besonderer Vorteil der Erfindung besteht darin, dass die Erinnerungszeiten einerseits automatisch ermittelt werden können, andererseits aber eine besonders flexible Anpassung an den Lebensrhythmus des jeweiligen erreicht wird. Insbesondere relativ junge und unabhängige Diabetiker haben - bedingt durch berufliche und persönliche von Tag zu Tag unterschiedliche Planungen - oft einen sehr unregelmäßigen Tagesablauf. Im Rahmen der Erfindung wurde festgestellt, dass in solchen Fällen eine Erinnerungszeitenermittlung, die sich an medizinischen Messergebnissen orientiert, nicht zu befriedigenden Ergebnissen führt. Die Erfindung ermöglicht es, verschiedene Ereigniszeitdaten flexibel im Rahmen des Erinnerungszeitermittlungsalgorithmus zu kombinieren, und dadurch die Erinnerungszeiten an den individuellen Lebensrhythmus des Benutzers anzupassen.

Um dies zu optimieren, werden vorzugsweise unterschiedliche Erinnerungszeitermittlungsalgorithmen einerseits für Arbeitstage und andererseits für arbeitsfreie Tage verwendet.

Eine flexible Anpassung der Erinnerungszeiten wird dadurch gefördert, dass in einem Erinnerungszeitermittlungsalgorithmus eine Ereigniszeit als nichtstatischer Bezugspunkt (floating reference point) benutzt wird. Insbesondere eignen sich hierzu die Nahrungsaufnahmen. Es kann vorteilhaft sein, innerhalb des Erinnerungszeitermittlungsalgorithmus für eine Mehrzahl von Tagen die zeitlichen Abstände zwischen einer Nahrungsaufnahme (beispielsweise dem Mittagessen) und der nächsten darauffolgenden Messung der Glucosekonzentration zu bestimmen und ein Erinnerungssignal jeweils zu einem Zeitpunkt zu erzeugen, der in einer Beziehung zu der entsprechenden Nahrungsaufnahme (z.B. Mittagessen) an dem jeweiligen Tag steht. Im einfachsten Fall könnte die Erinnerung jeweils in einem zeitlichen Abstand zu dem Mittagessen erfolgen, der dem Mittelwert der entsprechenden zeitlichen Abstände der Blutglucosemessungen von dem Mittagessen an den vorausgehenden Tagen entspricht. Der Mittelwert ist selbstverständlich nur ein besonders einfaches Beispiel. Es stehen mathematische Prozeduren zur Verfügung, die eine bessere Adaption unter Berücksichtigung mehrerer Einflussgrößen, also insbesondere mehrerer Ereignistypen und der entsprechenden Ereigniszeitdaten ermöglichen. Jedenfalls zeigt das Beispiel, dass die Verwendung eines nicht statischen Bezugspunktes besondere Vorteile im Hinblick auf die flexible Anpassung an die Gewohnheiten des Benutzers hat, weil er nicht zu einer mehr oder wenigen festen Tageszeit, sondern zu einem seinem persönlichen Tagesablauf entsprechenden Zeitpunkt an die Durchführung einer Glucosemessung erinnert wird. Dabei kann der zeitliche Abstand zwischen der als nicht statischer Bezugspunkt verwendeten Ereigniszeit und der Erinnerungszeit beliebige Werte annehmen, insbesondere als auch Null sein, so dass die Erinnerung gleichzeitig mit dem Eintreten des als Bezugspunkt verwendeten Ereignisses erfolgt

Insofern unterscheidet sich die Erfindung grundlegend von der WO 2006/037802 A2, die ein Verfahren und System zum Selbstmanagement einer Krankheit beschreibt, wobei es primär um die zeitlich und hinsichtlich des Volumens gewünschte Dosierung der bei einem Diabetiker erforderlichen Insulininjektionen geht. Im Gegensatz zu der Erfindung wird dort nicht das Ziel verfolgt, Erinnerungszeiten an die Gewohnheiten des Benutzers derart anzupassen, dass er nicht zu einer mehr oder weniger festen Tageszeit, sondern zu einem seinem persönlichen Tagesablauf entsprechenden Zeitpunkt, an die erforderliche Handlung erinnert wird. Vielmehr wird durch das in dem Dokument beschriebene Verfahren angestrebt, den Benutzer zu einem gewünschten standardisierten Verhalten zu bewegen. Es wird eine Änderung seines Verhaltens in diesem Sinne angestrebt Zu diesem Zweck werden seine bisherigen Gewohnheiten als sogenannte "strongest habits" ermittelt, wobei die Erinnerungszeit nicht an die Gewohnheiten des Benutzers angepasst, sondern stattdessen seine Gewohnheiten so modifiziert werden sollen, dass sie den "best practice rules of lifestyle, eating habits, etc." entsprechen.

Die Erfindung ermöglicht es, Erinnerungszeitpunkte zu ermitteln, die individuell an den Tagesrhythmus eines Benutzers angepasst sind und außerdem auch von Ereignissen, die für den zeitlichen Verlauf der Blutglucosekonzentration wichtig sind, beispielsweise Nahrungsaufnahmen, Insulingaben oder körperlicher Aktivität, abhängen.

Die Ermittlung von Erinnerungszeiten im Rahmen der Erfindung bezieht sich nicht nur auf notwendige Glucoseanalysen. Vielmehr können gemäß der Erfindung auch andere Erinnerungszeiten ermittelt werden, insbesondere können Erinnerungssignale erzeugt werden, mit denen an Insulingaben und/oder Nahrungsaufnahmen erinnert wird. Besonders günstig ist dabei, wenn mit der Signaleinrichtung unterschiedliche Erinnerungssignale erzeugt werden können. Generell kommen in erster Linie akustische Erinnerungssignale in Betracht, jedoch können auch visuelle Erinnerungssignale (beispielsweise Anzeigen auf einem Display) oder haptische Erinnerungssignale (z.B. Vibrationen) verwendet werden.

Beispielsweise kann von der Steuer- und Auswerteeinrichtung eine erste Klasse von Erinnerungszeiten ermittelt werden, zu denen die Signaleinrichtung ein Erinnerungssignal eines ersten Typs erzeugt, um an eine Messung der Blutglucosekonzentration zu erinnern, und zusätzlich eine zweite Klasse von Erinnerungszeiten ermittelt werden, zu denen von der Signaleinrichtung ein Erinnerungssignal eines zweiten Typs erzeugt wird, um an Insulininjektionen, Nahrungsaufnahmen und/oder sportliche Betätigung zu erinnern.

Die Erfindung wird nachfolgend anhand eines Ausführungsbeispiels unter Bezugnahme auf die beigefügten Zeichnungen näher erläutert. Die darin dargestellten Besonderheiten können einzeln oder in Kombination verwendet werden, um bevorzugte Ausgestaltungen zu schaffen. Es zeigen:
- Fig. 1: eine perspektivische Ansicht eines Glucoseanalysegeräts und
- Fig. 2: ein Blockschaltbild des in Figur 1 gezeigten Glucoseanalyse- geräts.

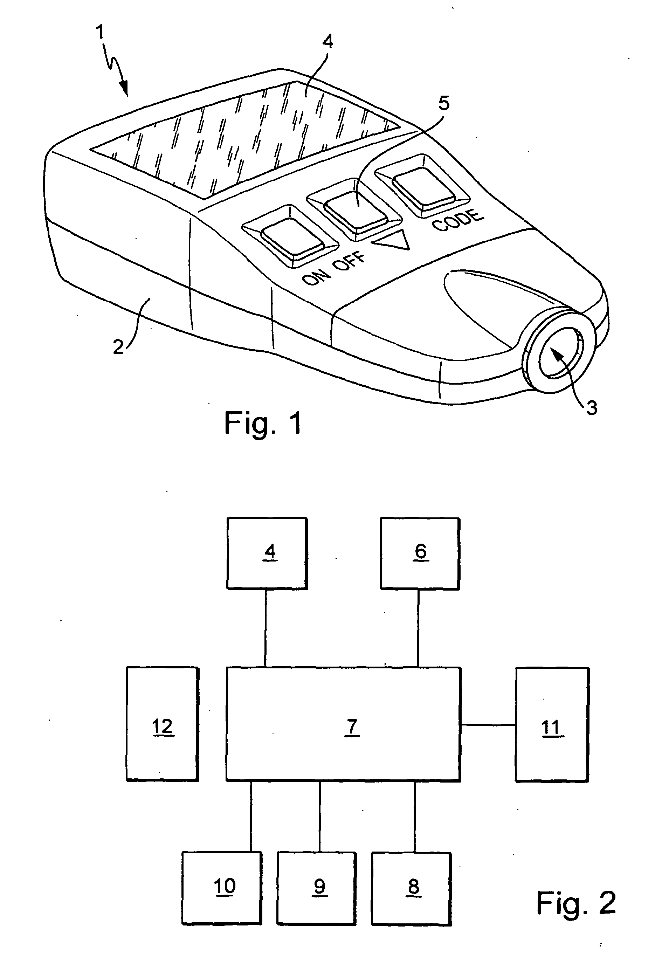

Das in Figur 1 gezeigte Glucoseanalysegerät 1 entspricht in seiner äußeren Erscheinung bekannten Geräten. Zum Anzeigen von Messwerten dient eine Anzeigeeinrichtung 4 in Form eines Displays. Zum Betätigen des Analysegeräts 1 und/oder für Sonderfunktionen sind Bedienungselemente 5 in Form von Tasten vorhanden. In dem Gerät befindet sich eine Lanzette (nicht dargestellt), mit der in einem Körperteil, beispielsweise einem Finger, der an eine Öffnung 3 des Gehäuses 2 angelegt wird, eine Einstichwunde erzeugt werden kann. Das Gerät 1 nimmt selbsttätig aus der Einstichwunde austretendes Blut auf und ermittelt mit einer Messeinrichtung (siehe Figur 2) einen Blutglucosekonzentrationswert.

Das Glucoseanalysegerät 1 ist mit einer netzunabhängigen Stromquelle (nicht dargestellt), beispielsweise handelsüblichen Batterien, ausgerüstet, mit der die einzelnen Komponenten gespeist werden. Die entsprechenden Komponenten des Glucoseanalysegeräts 1 sind bekannt und bedürfen deshalb keiner näheren Erläuterung.

In Figur 2 ist schematisch ein Blockschaltbild des Glucoseanalysegeräts 1 dargestellt. Der Glucosegehalt einer Blutprobe wird mit einer Messeinrichtung 6, die an eine Steuer- und Auswerteeinrichtung 7 angeschlossen ist, ermittelt. Als "Messeinrichtung" wird hier die Gesamtheit aller Komponenten bezeichnet, die in dem Analysegerät dazu dienen, eine für die Analyse charakteristische physikalische Messgröße (normalerweise an einem Analyseelement) zu messen und daraus die gewünschte Glucosekonzentration zu ermitteln. Eine nähere Erläuterung ist nicht erforderlich, weil zahlreiche derartige Messeinrichtungen bekannt sind und es auf die Art des analytischen Verfahrens nicht ankommt.

Die Steuer- und Auswerteeinrichtung 7 enthält einen Mikroprozessor. Ermittelte Glucosekonzentrationswerte werden in einem nichtflüchtigen Datenspeicher 8 gespeichert und mit der Anzeigeeinrichtung 4 einem Benutzer angezeigt.

Das Glucoseanalysegerät 1 verfügt ferner über eine interne Zeitmesseinrichtung 9, die beispielsweise einen Taktgeber der Steuer- und Auswerteeinrichtung 7 benutzt. Eine Signaleinrichtung 10 dient dazu, einen Benutzer, beispielsweise durch einen Signalton, an das Durchführen einer Blutglucosemessung zu erinnern.

In dem Datenspeicher 8 werden als Ereigniszeiten zusätzlich zu den gemessenen Glucosekonzentrationswerten die Messzeiten, d.h. die Uhrzeiten, gespeichert, zu denen Glucosekonzentrationswerte gemessen wurden. Von der Steuer- und Auswerteeinrichtung 7 werden auf der Grundlage der gespeicherten Ereigniszeiten Erinnerungszeiten ermittelt, zu denen die Signaleinrichtung 10 von der Steuer- und Auswerteeinrichtung 7 betätigt wird. Diese Erinnerungszeiten werden von der Steuer- und Auswerteeinrichtung 7 mit einem Verfahren (Algorithmus) ermittelt, das im Folgenden erläutert wird. Dabei wird zunächst ein sehr einfaches Verfahren beschrieben, bei dem ausschließlich die Ereigniszeitdaten von Glucoseanalysen berücksichtigt werden.

Bei der erstmaligen Inbetriebnahme des Glucoseanalysegeräts 1 sind noch keine Ereigniszeiten, d.h. Zeiten, zu denen Blutglucosemessungen durchgeführt wurden, gespeichert. Die Steuer- und Auswerteeinrichtung 7 beginnt deshalb mit ihrer eigentlichen Tätigkeit erst an dem zweiten Tag nach der erstmaligen Inbetriebnahme und greift dabei auf die gespeicherten Ereigniszeiten zurück, zu denen an dem ersten Tag Blutglucosemessungen durchgeführt wurden. An dem zweiten Betriebstag werden die gespeicherten Ereigniszeiten des ersten Betriebstags als Erinnerungszeiten verwendet. Die Signaleinrichtung 10 wird deshalb an dem zweiten Betriebstag von der Steuer- und Auswerteeinrichtung 7 genau zu jenen Tageszeiten betätigt, zu denen an dem ersten Betriebstag Blutglucosemessungen durchgeführt wurden.

Für den dritten und jeden weiteren Betriebstag werden die Erinnerungszeiten jeweils neu berechnet. Dazu werden jene Ereigniszeiten betrachtet, die in einem vorgegebenen Zeitintervall um die zuletzt verwendete Erinnerungszeit liegen. Aus diesen Ereigniszeiten wird die neue Erinnerungszeit als Mittelwert berechnet.

Die Größe dieses Zeitintervalls ist so zu wählen, dass beispielsweise Ereigniszeiten der ersten Messung eines Tages bei der Berechnung der Erinnerungszeit für die zweite Messung des gleichen Tages unberücksichtigt bleiben. Das Zeitintervall sollte also so klein sein, dass in ihm zur Berechnung der Erinnerungszeit für die n-te Messung eines Tages nur Ereigniszeiten von n-ten Messungen vergangener Tage enthalten sind. Bevorzugt umfaßt das Zeitintervall etwa ein bis zwei Stunden.

Bevorzugt werden bei der Berechnung der Erinnerungszeiten nur Ereigniszeitdaten eines begrenzten Zeitraums von beispielsweise zwei Wochen berücksichtigt, so dass eine Anpassung an einen geänderten Tagesrhythmus in überschaubarer Zeit erfolgt. Je kürzer dieser Zeitraum gewählt wird, desto schneller entsprechen die Erinnerungszeiten geänderten Lebensgewohnheiten. Dennoch darf dieser Zeitraum nicht zu kurz gewählt werden, da sonst Messungen, die aus besonderem Anlass zu ungewohnten Zeiten durchgeführt wurden, einen unerwünscht großen Einfluß auf die Berechnung der Erinnerungszeiten haben. Günstig ist es, bei der Berechnung der Erinnerungszeiten jeweils die Ereigniszeitdaten der vorhergehenden 4 bis 14 Tage zu verwenden.

In diesem Zusammenhang ist es vorteilhaft, wenn der Benutzer die Möglichkeit hat, gespeicherte Ereigniszeitdaten zu löschen oder für den ersten Tag Erinnerungszeiten einzuprogrammieren. Zu diesem Zweck kann die Anzeigeeinrichtung 4 zum Abbilden eines Bedienungsmenüs genutzt werden, aus dem mit den Bedienungselementen 5 geeignete Funktionen ausgewählt werden.

Wie erwähnt, wurde vorstehend ein relativ einfacher Erinnerungszeitermittlungsalgorithmus mit einigen Varianten erläutert. In Anbetracht der heute auch in kleinen batteriebetriebenen Geräten zur Verfügung stehenden Prozessor- und Datenspeicherkapazitäten können sehr viel aufwendigere Algorithmen eingesetzt werden. Eine detaillierte Erörterung ist wegen der Vielzahl der möglichen Varianten nicht möglich. Sie ist auch nicht notwendig, da die mathematischen Verfahren bekannt und weitgehend als kommerzielle Programmbausteine erhältlich sind. Die mit der Erfindung und deren erläuterten bevorzugten Ausgestaltungen verbundenen Vorteile lassen sich mit unterschiedlichen Varianten solcher kommerziell erhältlicher Algorithmen erreichen.

Wie bereits erläutert wurde, ist die Erfindung nicht auf Messungen der Glucosekonzentration als Ereignistyp beschränkt. Vielmehr werden vorzugsweise Ereigniszeitdaten anderer, insbesondere auch externer, Ereignistypen verwendet. Sie können entweder über eine Eingabeeinrichtung 11 manuell oder automatisch mittels eines Ereignissensors 12 an die Steuer- und Auswerteeinrichtung 7 übermittelt werden.

Bevorzugt bleiben bei der Ermittlung der Erinnerungszeiten Wiederholungsmessungen unberücksichtigt, die beispielsweise durchgeführt wurden, um auffällige Messwerte mit besonders hohen oder niedrigen Glucosekonzentrationswerten zu überprüfen. In diesem Zusammenhang ist es bevorzugt, dass die Steuer- und Auswerteeinrichtung jeweils ermittelte Glucosekonzentrationswerte mit historischen Messwerten vergleicht und bei auffälligen Messwerten nach einer vorgegebenen Zeit von beispielsweise 20 Minuten selbsttätig an eine Wiederholungsmessung erinnert.

Ein erhöhter Benutzerkomfort läßt sich dadurch erreichen, dass für Arbeitstage ein anderer Erinnerungszeitermittlungsalgorithmus verwendet wird als für arbeitsfreie Tage. Die meisten Menschen haben an Arbeitstagen einen anderen Tagesrhythmus als an arbeitsfreien Tagen (Urlaubstagen, Feiertagen und Wochenenden). Am einfachsten erfolgt die Umschaltung des Erinnerungszeitermittlungsalgorithmus für Arbeits- und arbeitsfreie Tage dadurch, dass der Benutzer durch einen Tastendruck dem Gerät 1 mitteilt, ob der heutige Tag ein arbeitsfreier Tag ist. Auch für andere besondere Lebenssituationen, beispielsweise für den Fall einer Erkrankung, kann in ähnlicher Weise ein spezieller Erinnerungszeitermittlungsalgorithmus vorgesehen sein.

Bevorzugt wird die Signaleinrichtung 10 zusätzlich dazu verwendet, einen Benutzer an eine Basalinjektion, d.h. eine Insulininjektion zur Deckung seines Grundbedarfs, zu erinnern. Die entsprechenden Erinnerungszeiten für Basalinjektionen können ebenso wie die Erinnerungszeiten für Blutglucosemessungen mit einem Erinnerungszeitermittlungsalgorithmus ermittelt werden. Dazu ist es lediglich erforderlich, dass dem Gerät der Zeitpunkt einer Basalinjektion mitgeteilt wird. Im einfachsten Fall kann dies durch einen Tastendruck geschehen, so dass in dem Datenspeicher 8 die aktuelle Zeit zum Zeitpunkt des Tastendrucks als Ereigniszeit für eine Basalinjektion gespeichert wird. Bevorzugt wird mit der Signaleinrichtung 10 zum Erinnern an eine Blutglucosemessung ein anderes Signal als zum Erinnern an eine Basalinjektion erzeugt. Für das menschliche Ohr leicht unterscheidbare Erinnerungsignale können beispielsweise durch unterschiedliche Tonhöhen oder Tonfolgen erzeugt werden.

Die Steuer- und Auswerteeinrichtung 7 kann auch dazu verwendet werden, durch Auswerten von in dem Datenspeicher 8 gespeicherten Messwerten der Blutglucosekonzentration unter Berücksichtigung von Ereigniszeitdaten (z.B. über Insulingaben, Nahrungsaufnahmen und körperliche Aktivitäten) auch automatisch an Korrekturinsulingaben, Nahrungsaufnahmen oder körperliche Aktivitäten zu erinnern.

## Patentansprüche

1. Glucoseanalysegerät für Diabetiker, umfassend:
eine Messeinrichtung (6) zum Ermitteln von Glucosekonzentrationswerten,
eine Anzeigeeinrichtung (4) zum Anzeigen von Glucosekonzentrationswerten,
eine Signaleinrichtung (10) zum Erzeugen eines Erinnerungssignals und
eine Steuer- und Auswerteeinrichtung (7), die einen Prozessor und einen Datenspeicher (8) einschließt und mittels der Erinnerungszeiten ermittelt werden, zu denen die Signaleinrichtung (10) betätigt wird,
wobei
in dem Datenspeicher (8) Ereigniszeitdaten gespeichert werden, welche Informationen enthalten über Ereignisse, die zu mindestens einem vordefinierten Ereignistyp im Leben eines Benutzers des Glucoseanalysegerätes gehören, wobei der mindestens eine vordefinierte Ereignistyp den Typ Nahrungsaufnahme einschließt und Informationen über den Ereignistyp und über die Zeit der Ereignisse gespeichert werden,
die Erinnerungszeiten mittels eines Erinnerungszeitermittlungsalgorithmus unter Verwendung von Ereigniszeitdaten, einschließlich des Ereignistyps und der Zeit des Ereignisses, aus mehreren Tagen ermittelt werden und
eine Ereigniszeit eines Ereignisses, das zu einem vorbestimmten Ereignistyp gehört, insbesondere die Zeit einer Nahrungsaufnahme, in dem Erinnerungszeitermittlungsalgorithmus als nichtstatischer Bezugspunkt benutzt wird, um eine Erinnerungszeit mittels des zeitlichen Abstandes zwischen der als nichtstatischer Bezugspunkt verwendeten Ereigniszeit und der Erinnerungszeit zu berechnen.

2. Glucoseanalysegerät nach Anspruch 1, **dadurch gekennzeichnet, dass** in dem Datenspeicher während der Benutzung des Gerätes Ereigniszeitdaten für mindestens 5, bevorzugt mindestens 8, besonders bevorzugt mindestens 12 Tage akkumuliert werden.

3. Glucoseanalysegerät nach einem der Ansprüche 1 oder 2, **dadurch gekennzeichnet, dass** interne Ereignisse des Glucoseanalysegerätes, insbesondere Messungen der Glucosekonzentration, einen vordefinierten internen Ereignistyp der Ereigniszeitdaten bilden.

4. Glucoseanalysegerät nach Anspruch 3, **dadurch gekennzeichnet, dass** der Erinnerungszeitermittlungsalgorithmus einen Funktionsschritt enthält, durch den das Betätigen der Signaleinrichtung (10) zu einer ermittelten Erinnerungszeit unterbleibt, wenn in einem vorgegebenen Karenzzeitintervall von vorzugsweise mindestens 20 Minuten vor der Erinnerungszeit eine Messung der Glucosekonzentration durchgeführt wurde.

5. Glucoseanalysegerät nach einem der vorhergehenden Ansprüche, **dadurch gekennzeichnet, dass** externe Ereignisse außerhalb des Glucoseanalysegerätes, insbesondere Ruheperioden des Benutzers, Insulingaben oder körperliche Aktivitätsperioden des Benutzers einen vordefinierten externen Ereignistyp der Ereigniszeitdaten bilden.

6. Glucoseanalysegerät nach Anspruch 5, **dadurch gekennzeichnet, dass** Ereigniszeitdaten eines externen Ereignistyps automatisch mittels eines Ereignissensors ermittelt werden.

7. Glucoseanalysegerät nach Anspruch 5, **dadurch gekennzeichnet, dass** Ereigniszeitdaten eines externen Ereignistyps mittels einer Eingabeeinrichtung manuell eingegeben werden können.

8. Glucoseanalysegerät nach einem der vorhergehenden Ansprüche, **dadurch gekennzeichnet, dass** der Erinnerungszeitermittlungsalgorithmus ein Mustererkennungsverfahren einschließt.

9. Glucoseanalysegerät nach einem der vorhergehenden Ansprüche, **dadurch gekennzeichnet, dass** für Arbeitstage und für arbeitsfreie Tage die Erinnerungszeiten mittels unterschiedlicher Erinnerungszeitermittlungsalgorithmen ermittelt werden.

10. Glucoseanalysegerät nach einem der vorhergehenden Ansprüche, **dadurch gekennzeichnet, dass** der Datenspeicher (8) nichtflüchtig ist.

11. Glucoseanalysegerät nach einem der vorhergehenden Ansprüche, **dadurch gekennzeichnet, dass** von der Signaleinrichtung (10) akustische Erinnerungssignale erzeugt werden.

12. Glucoseanalysegerät nach einem der vorhergehenden Ansprüche, **dadurch gekennzeichnet, dass**
mittels der Steuer- und Auswerteeinrichtung (7) eine erste Klasse von Erinnerungszeiten ermittelt wird, zu denen von der Signaleinrichtung (10) ein Erinnerungssignal eines ersten Typs erzeugt wird, um an eine Messung der Blutglucosekonzentration zu erinnern, und
mittels der Steuer- und Auswerteeinrichtung (7) eine zweite Klasse von Erinnerungszeiten ermittelt wird, zu denen von der Signaleinrichtung (10) ein Erinnerungssignal eines zweiten Typs erzeugt wird, um an eine Insulininjektion zu erinnern.

## Claims

1. Glucose analysis instrument for diabetics, comprising:
a measuring device (6) for determining glucose concentration values,
a displaying device (4) for displaying glucose concentration values,
a signaling device (10) for generating a reminder signal, and
a control and evaluation device (7) which comprises a processor and a data memory (8) and is used to determine reminder times at which the signaling device (10) is actuated,
wherein
event time data are stored in the data memory (8), said event time data containing information on events which belong to at least one predefined event type in the life of a user of the glucose analysis instrument, wherein the at least one predefined event type includes the food intake type and wherein information on the event type and the event time are stored,
the reminder times are determined by means of a reminder time determination algorithm using event time data, including event type and event time, from a plurality of days, and
the event time of an event belonging to a predefined event type, in particular the time of a food intake, is used as a floating reference point in the reminder time determination algorithm, in order to calculate a reminder time by means of the difference in time between the event time which is used as a floating reference point and the reminder time.

2. Glucose analysis instrument according to claim 1, **characterized in that** event time data are accumulated in the data memory for at least 5, preferably at least 8, particularly preferably at least 12 days during the use of the instrument.

3. Glucose analysis instrument according to any one of the claims 1 or 2, **characterized in that** internal events of the glucose analysis instrument, in particular glucose concentration measurements, are a pre-defined internal event type of the event time data.

4. Glucose analysis instrument according to claim 3, **characterized in that** the reminder time determination algorithm comprises a functional step that prevents the actuation of the signaling device (10) at a reminder time if a glucose concentration measurement was carried out in a pre-set waiting period of preferably at least 20 minutes prior to the reminder time.

5. Glucose analysis instrument according to any one of the preceding claims, **characterized in that** events external to the glucose analysis instrument, in particular food intakes of the user, resting periods of the user, insulin administrations or periods of physical activity of the user, are a predefined external event type of the event time data.

6. Glucose analysis instrument according to claim 5, **characterized in that** information about the occurrence of an external event type is generated automatically by means of an event sensor.

7. Glucose analysis instrument according to claim 5, **characterized in that** information about the occurrence of an external event type can be entered manually by means of an input device.

8. Glucose analysis instrument according to any one of the preceding claims, **characterized in that** the reminder time determination algorithm comprises a pattern recognition method.

9. Glucose analysis instrument according to any one of the preceding claims, **characterized in that** the reminder times for working days and for non-working days are determined by means of different reminder time determination algorithms.

10. Glucose analysis instrument according to any one of the preceding claims, **characterized in that** the data memory (8) is non-volatile.

11. Glucose analysis instrument according to any one of the preceding claims, **characterized in that** the signaling device (10) generates acoustic reminder signals.

12. Glucose analysis instrument according to any one of the preceding claims, **characterized in that**
the control and evaluation device (7) determines a first class of reminder times at which the signaling device (10) generates a first type of reminder signal as a reminder of a blood glucose concentration measurement, and
the control and evaluation device (7) determines a second class of reminder times at which the signaling device (10) generates a second type of reminder signal as a reminder of an insulin injection.

## Revendications

1. Appareil d'analyse de glucose pour diabétiques, comprenant
un dispositif de mesure (6) pour la détermination de valeurs de concentration de glucose,
un dispositif d'affichage (4) pour l'affichage de valeurs de concentration de glucose,
un dispositif de signalisation (10) pour générer un signal de rappel et
un dispositif de commande et d'analyse (7), qui inclut un processeur et une mémoire de données (8) et qui permet de déterminer des heures de rappel, où le dispositif de signalisation (10) est actionné,
des données d'heure d'événement étant mémorisées dans la mémoire de données (8), lesquelles contiennent des informations, qui font partie d'au moins un type d'événement prédéfini dans la vie d'un utilisateur de l'appareil d'analyse de glucose, le au moins un type d'événement prédéfini incluant le type absorption de nourriture et des informations concernant le type d'événement et concernant l'heure de l'événement étant mémorisées,
les heures de rappel étant déterminées au moyen d'un algorithme de détermination d'heure de rappel avec l'utilisation de données d'heure d'événement, y compris le type d'événement et l'heure de l'événement, à partir de plusieurs jours et
une heure d'événement d'un événement, qui fait partie d'un type d'événement prédéfini, en particulier l'heure d'une absorption de nourriture, étant utilisée dans l'algorithme de détermination d'heure de rappel comme point de référence non statique, afin de calculer une heure de rappel au moyen de l'écart de temps entre l'heure d'événement utilisée comme point de référence non statique et l'heure de rappel.

2. Appareil d'analyse de glucose selon la revendication 1, **caractérisé en ce que** des données d'heure d'événement pour au moins 5, de préférence au moins 8, avec une préférence particulière au moins 12 jours sont accumulées dans la mémoire de données pendant l'utilisation de l'appareil.

3. Appareil d'analyse de glucose selon l'une des revendications 1 ou 2, **caractérisé en ce que** des événements internes de l'appareil d'analyse de glucose, en particulier des mesures de la concentration de glucose, forment un type d'événement interne prédéfini des données d'heure d'événement.

4. Appareil d'analyse de glucose selon la revendication 3, **caractérisé en ce que** l'algorithme de détermination d'heure de rappel contient une étape de fonction, par laquelle l'actionnement du dispositif de signalisation (10) n'a pas lieu à une heure de rappel déterminée, lorsque, dans un intervalle de temps de carence prédéfini de préférence d'au moins 20 minutes avant l'heure de rappel, une mesure de la concentration de glucose a été effectuée.

5. Appareil d'analyse de glucose selon l'une des revendications précédentes, **caractérisé en ce que** des événements externes en dehors de l'appareil d'analyse de glucose, en particulier des périodes de repos de l'utilisateur, des indications d'insuline ou des périodes d'activité physique de l'utilisateur forment un type d'événement externe prédéfini des données de temps d'événement.

6. Appareil d'analyse de glucose selon la revendication 5, **caractérisé en ce que** des données d'heure d'événement d'un type d'événement externe sont déterminées automatiquement au moyen d'un capteur d'événement.

7. Appareil d'analyse de glucose selon la revendication 5, **caractérisé en ce que** des données d'heure d'événement d'un type d'événement externe peuvent être entrées manuellement au moyen d'un dispositif d'entrée.

8. Appareil d'analyse de glucose selon l'une des revendications précédentes, **caractérisé en ce que** l'algorithme de détermination d'heure de rappel inclut un procédé de reconnaissance de modèle.

9. Appareil d'analyse de glucose selon l'une des revendications précédentes, **caractérisé en ce que** les heures de rappel sont déterminées pour des jours de travail et pour des jours sans travail au moyen de différents algorithmes de détermination d'heure de rappel.

10. Appareil d'analyse de glucose selon l'une des revendications précédentes, **caractérisé en ce que** la mémoire de données (8) n'est pas volatile.

11. Appareil d'analyse de glucose selon l'une des revendications précédentes, **caractérisé en ce que** des signaux de rappel acoustiques sont générés par le dispositif de signalisation (10).

12. Appareil d'analyse de glucose selon l'une des revendications précédentes, **caractérisé**
**en ce qu'**une première classe d'heures de rappel, où le dispositif de signalisation (10) génère un signal de rappel d'un premier type pour rappeler une mesure de la concentration de glucose du sang, est déterminée au moyen du dispositif de commande et d'analyse (7), et
une seconde classe d'heures de rappel, où le dispositif de signalisation (10) génère un signal de rappel d'un second type pour rappeler une injection d'insuline, est déterminée au moyen du dispositif de commande et d'analyse (7).
